# EUROPEAN PATENT APPLICATION

(11) **EP 4 838 220 A1**
(43) Date of publication of application: **11.11.2026**
(21) Application number: 25177414.7
(22) Date of filing: 19.05.2025
(51) Int. Cl.: G01N 33/497, G06Q 50/00

(54) **DEVICE AND SYSTEM FOR DETERMINING A BREATH ALCOHOL CONCENTRATION OF A USER AND METHOD THEREOF**

(30) Priority: 06.05.2025 PT 2025120244
(71) Applicant: TIAGO OLIVEIRA MONTEIRO UNIPESSOAL LDA, 4800-167 Guimaraes (PT)
(72) Inventor: OLIVEIRA MONTEIRO, TIAGO, 4800-167 GUIMARÃES (PT)
(74) Representative: Patentree

(57) **Abstract**

The present disclosure provides a digital breathalyzer device, system, and method for accurately determining a user's breath alcohol concentration (BrAC). This device comprises a robust frame, a high-precision breathalyzer sensor, a disposable straw dispensing mechanism, and a multifunctional tactile screen capable of presenting diverse interactive and promotional content. It integrates secure payment processing, advanced user identification methods, and dynamic QR code generation for streamlined interaction and enhanced user experience.

The present disclosure comprises extensive real-time data collection, secure transmission, and comprehensive analytics via a centralized Global Manager Server (GMS). The GMS facilitates predictive maintenance, automated calibration alerts, and detailed monitoring of device status. Additionally, it employs advanced machine learning algorithms for predictive analytics, providing personalized, context-sensitive feedback to users, including targeted educational content and proactive mobility recommendations integrated seamlessly with local transportation services. The device's interactive functionalities extend to immersive augmented reality experiences, enhancing user awareness about alcohol consumption effects. It features robust anti-vandalism measures, structural integrity enhancements, and environmental sustainability features such as photovoltaic energy integration and components. Furthermore, the system supports multi-language interactions and accessibility features, enabling universal usability across diverse demographics.

Real-time event analytics delivered by the GMS offer venue operators and urban administrators detailed insights into user behavior patterns, alcohol consumption trends, device usage metrics, and peak activity periods, facilitating informed decision-making and improved public safety strategies. This combination of advanced technological features and comprehensive data analysis capabilities positions the disclosed device as a solution for enhancing individual safety, promoting responsible behavior, and significantly contributing to urban safety and smart city initiatives.

## Description

### TECHNICAL FIELD

The present disclosure relates to a device and system for detecting the level of alcohol of the breath of a user. Particularly, it relates to a device and method of detecting and measuring the level of alcohol of the breath of a user and inform said user of the result. The present disclosure relates to a digital breathalyzer device designed to accurately detect and measure breath alcohol concentration (BrAC) levels of users. Specifically, this device integrates advanced sensor technologies, sophisticated real-time data processing, and analytical capabilities to manage and interpret various types of data and user information. Its multifunctional tactile screen supports diverse interactive features, including audiovisual advertising, dynamic QR code generation, and interactive questionnaires with touch-sensitive questions, wherein the responses are securely stored and analyzed by the integrated system. Additionally, the device supports enhanced data encryption and privacy, predictive maintenance for sensor calibration, personalized user feedback, integrated urban mobility recommendations, robust anti-vandalism features, sustainable operational components, real-time analytics for event management, augmented reality interactions, and comprehensive multi-language accessibility features, thereby providing a comprehensive solution for individual safety and societal benefit.

### BACKGROUND

Law enforcement agencies widely use conventional devices for measuring alcohol level of a breath of a user (e.g. breathalyzers) to measure breath alcohol concentration (BrAC) for road safety. However, these devices are typically limited to providing a numerical BrAC value, offering users no additional context or guidance. With increasing concerns about road safety and the societal impact of alcohol-related accidents, there is a growing need for more advanced solutions that detect alcohol levels before driving, and actively engage with users to promote responsible behaviour.

The development of technologies for monitoring alcohol consumption in public contexts and events has mainly focused on traditional devices that, although effective in essential measurements, lack advanced connectivity and digital integration capabilities [1]. The evolution of the Internet of Things (IoT) and Artificial Intelligence (AI) has opened up new possibilities for connected devices, providing continuous, real-time monitoring and enabling the implementation of personalized feedback [2] and predictive analytics [3]. However, the application of these technologies to alcohol monitoring devices is still in its infancy and faces technical and privacy challenges.

Several related solutions address the detection of breath alcohol concentration, including traditional breathalyzers, portable commercial devices [4] and vehicle-integrated systems. Traditional breathalyzers, primarily used by law enforcement, provide accurate BrAC measurements but lack interactive feedback or preventive features.

Portable devices offer personal BrAC monitoring but are often limited in precision and need more integration with broader safety solutions [5]. Document US11703489B2 is an example of a monitoring breathalyzer that is comprised of an alcohol sensor, a processing unit, and a screen. The alcohol sensor is calibrated with a sample measurement from the user before drinking (reference point). Measurements of BrAC are made in comparison with the reference point. However, the device lacks precision and broader integration with other solutions.

Vehicle-integrated systems, such as ignition interlocks, prevent driving under the influence but fail to educate or provide alternatives [6].

Document US10421358B1 presents a device with an integrated breathalyzer that functions as a transportation safety device, as it can communicate with a vehicle and prevent its ignition. This solution fails to provide a precise measure.

Document KR102085489B1 offers and alternative solution for the prevention of alcohol-related road accidents in the context of transportation services. It describes a management system comprising a breathalyzer management terminal for installation in an office, a network and a server. The breathalyzer management terminal recognizes the worker through fingerprint reading and analyses his BrAC. The conformity/non-conformity information is transmitted through the network and stored in the server for analysis and management of the drivers by the company. This system lacks the functionality to interact with the drivers by the test results and prevent them from driving. Additionally, this solution is designed for closed environments (inside companies) and there's a need for comprehensive solutions that reach the majority of the driving population.

These technologies address isolated aspects of alcohol-related safety but do not offer a comprehensive solution. The primary limitations include the need for real-time user engagement, connectivity to digital platforms, and integration with urban planning or public safety strategies.

Moreover, recent studies show that integrating intelligent sensors and communication protocols between devices is an emerging trend in smart cities [7], where interconnected solutions aim to improve public safety and support urban planning. In the specific context of security at events and public establishments, connected devices can reduce incident response time [8] and provide aggregated data that helps create preventative health and safety policies [9].

Despite this, alcohol monitoring devices on the market still need to fully exploit the potential of data for feedback adaptation and the ability to transmit data to central networks, representing a significant gap in interconnection with city ecosystems.

Also, nightlife events and establishments, such as nightclubs, bars and festivals, play a vital role in urban culture and economic activity [10]. However, these venues often face significant security challenges, including overcrowding, substance misuse, theft, and interpersonal conflicts [11]. Several state-of-the-art reviews explore the necessity and current advancements in digital security technologies tailored for nightlife environments [12].

Moreover, collaborative frameworks between city authorities, technology providers [13] and venue operators will be critical for establishing standardized practices and fostering innovation [14].

These facts are disclosed in order to illustrate the technical problem addressed by the present disclosure.

### GENERAL DESCRIPTION

The present disclosure relates to a device and system for detecting the level of alcohol of the breath of a user. Particularly, it relates to a device and method of detecting and measuring the level of alcohol of the breath of a user and inform said user of the result.

It is disclosed a device for determining a breath alcohol concentration of a user comprising: a frame for supporting the device; a breathalyzer for detecting the breath alcohol concentration of the user; a disposable straw dispenser attached to the frame, for dispensing a disposable straw to perform the breath alcohol concentration detecting; a disposable straw reservoir containing disposable straws for feeding the disposable straw dispenser with straws; an aperture attached to the frame and connected to the breathalyzer for receiving the disposable straw for the user to blow onto; a tactile screen attached to the frame for displaying information to the user and for the user to input information; a payment terminal attached to the frame for receiving payments; and a computer processing unit connected to the breathalyzer, the disposable straw dispenser, the tactile screen and the payment processor, wherein said computer processing unit is configured to:
receive payment data from the payment terminal;
dispense the disposable straw from the disposable straw reservoir to the disposable straw dispenser;
acquire the breath alcohol concentration from the breathalyzer;
display the acquired breath alcohol concentration on the tactile screen; and display promotional and/or interactive content on the tactile screen based on the detected breath alcohol concentration.

In an embodiment, the device comprises a card reader for receiving an identity card of the user, wherein said card reader is electronically connected to the computer processing unit for identifying the user for which the acquired breath alcohol concentration is made.

In an embodiment, the device comprises a wireless internet router for transmitting information through the internet to a Global Manager Server, GMS, of the acquired breath alcohol concentration and, optionally, the user identification.

In an embodiment, the device comprises a Global Positioning, GPS, tracker, for tracking the location of the device.

In an embodiment, the device comprises a plurality of lights wherein the plurality of lights can be selected from: LEDs, incandescent lights or florescent lights.

In an embodiment, the device comprises a microcontroller data processor configured to change the color and/or intensity of the plurality of lights.

In an embodiment, the computer processing unit is further configured to send lighting commands to the microcontroller data processor, wherein said lighting commands include adjusting the color of the plurality of lights, and/or the intensity of the plurality of lights.

In an embodiment, the computer processing unit is also configured to calibrate the breathalyzer, to count the number of disposable straws in the disposable straw reservoir, and to send an alert to the Global Manager Server, GMS whenever the disposable straw reservoir contains a low amount of disposable straws.

In an embodiment, the device comprises one or more sound speakers, connected to the computer processing unit, for emitting sound and music in respect to the promotional and/or interactive content.

In an embodiment, the identity card of the user read by the card reader is selected from: a driver's license, a government issued identification card and/or a passport.

In an embodiment, the computer processing unit is configured for sending sound messages and/or sound recordings to the one or more sound speakers, synchronously with the displayed promotional and/or interactive content on the tactile screen.

In an embodiment, the displayed promotional and/or interactive content may be selected from: a questionnaire for the user to answer, a disclaimer regarding driving under the influence of alcohol, and a promotional content.

In an embodiment, the payment terminal is a contactless payment terminal.

It is also disclosed a system for determining a breath alcohol concentration of a user, comprising one or more devices for determining a breath alcohol concentration of a user, and a Global Manager Server, GMS, configured to:
receive the breath alcohol concentration of the user from the breathalyzer from one of the one or more devices,
send promotional and/or interactive content to the one or more devices, based on the detected breath alcohol concentration,
send an alert to each of the one or more devices, to perform breathalyzer calibration, and validate the currency payment type made by the user during the test.

It is also disclosed a method for determining a breath alcohol concentration of a user, comprising: sending the breath alcohol concentration from the breathlyzer of one of the one or more devices to the computer processing unit of the one of the one or more devices; forwarding the sent breath alcohol concentration from the computer processing unit of the one of the one or more devices to the Global Manager Server, GMS; processing the forwarded breath alcohol concentration by the GMS; and registering the processed breath alcohol concentration by the GMS.

In an embodiment, the method of communication with the GMS is by Message Queuing Telemetry Transport (MQTT) protocol, the method comprising using a publisher/subscriber model, in which the computer processing unit of said one or more devices acts as a publisher, sending the breath alcohol concentration data to a specific topic on a MQTT broker, the GMS subscribing to the specific topic for receiving data in real-time, and processing the forwarded breath alcohol concentration by:
extracting the forwarded breath alcohol concentration, and any associated metadata, from the one or more devices;
transforming the extracted data for uniform data formatting, anomaly removal, or adding context, in particular user identification, time stamps; and
loading into the GMS the transformed data.

It is also disclosed a computer processing unit configured for carrying out the steps of the method.

It is also disclosed a computer program comprising instructions which, when the program is executed by a computer, cause the computer to carry out the steps of the method.

It is also disclosed a computer-readable storage medium comprising instructions which, when executed by a computer, cause the computer to carry out the steps of the method, in particular the computer-readable storage medium being a non-transitory computer-readable storage medium.

The present disclosure relates to a smart and interactive digital breathalyzer device for detecting and measure alcohol level and management system. More specifically, it relates to a hybrid breathalyzer system for detecting and measuring breath alcohol levels and interact with the user with personalized feedback.

Integrating intelligent technologies and interactive feedback mechanisms present an opportunity to transform the traditional breathalyzer into a multifunctional device that enhances individual decision-making, while contributing to broader public safety and urban planning efforts. The increasing reliance on digital solutions offers a transformative opportunity to address the aforementioned nightlife event and establishment associated issues, namely overcrowding, substance misuse, theft, and interpersonal conflicts.

The disclosed smart digital device and system will fill in this gap by offering a multifunctional solution with possible integration with intelligent city networks, transmitting real-time data to central platforms and enabling advanced data analytics to promote citizen safety and education. Studies in predictive analysis and machine learning for public health data demonstrate that using these technologies can significantly improve the effectiveness of prevention and public safety initiatives.

The disclosed digital device and system are designed to improve the approach to alcohol detection and safety. Unlike traditional devices, which are limited to providing numerical breath alcohol concentration readings, this solution combines precise BrAC measurement with advanced technological features, including cloud-based data processing, personalized user feedback, educational content, and integration with mobility solutions, creating an intelligent and interactive solution for individual safety and societal benefit. By retaining the core functionality of disposable straws for BrAC measurement and enhancing it with interactive capabilities, the device provides real-time educational content and actionable recommendations. This combination creates a proactive tool that empowers users to make safer decisions and contributes to public safety initiatives and urban planning through aggregated data analysis. This solution bridges the gap between conventional breathalyzers and modern loT-enabled devices, offering a solution to reduce alcohol-related risks and promote responsible behaviour.

The present disclosure introduces a smart digital device that addresses the critical limitations of existing alcohol detection devices by offering a comprehensive and innovative solution. It retains the accuracy of traditional breathalyzers, while further comprising advanced features. Specifically, the device intends to educate users through interactive content, promote responsible behaviour and provide actionable recommendations to reduce risks, such as alternative transportation options.

In an embodiment, the device retains the functionality of traditional breathalysers, that use disposable straws for sample collection, but stands out with its integration with advanced processing units capable of analysing test data and metrics. These are then processed in a cloud environment and deliver educational content and recommendations, including mobility solutions like car-sharing, to reduce road accident risks. Combining alcohol detection with real-time interactivity bridges the gap between conventional breathalyzers and modern loT-integrated solutions, supporting public safety initiatives and urban planning through data-driven insights.

This proactive approach transforms the device (breathalyzer) from a reactive detection tool into a multifunctional device that supports individual and public safety. The present disclosure bridges the gap between conventional technologies and modern loT ecosystems, by generating valuable data for urban planning and policy development. Its compact and portable design ensures accessibility across various contexts, making it a scalable and impactful solution aligned with city frameworks.

Regarding data collection and processing, an embodiment of the discloses comprises an optional feature where quick and interactive questionnaires appear on the screen, when selected, for a user to answer. This mechanism enhances user engagement with the device and provides invaluable insights into user behaviours and preferences in various contexts.

Moreover, the proposed device can also integrate advanced identification (ID) verification and comprehensive incident reporting capabilities. This enhancement emphasizes security and operational efficiency across various environments, including nightclubs, stadiums, businesses, cruise ships, casinos, restaurants, events, and music festivals. This system provides valuable insights and tools for venue operators and users, promoting a safer and more enjoyable experience.

The level of development in monitoring alcohol consumption is typically limited to traditional and manual breathalyzers, which are only used by public security forces, with no similar solution available for the general public at events and establishments. Additionally, traditional breathalyzers only provide basic measurements of blood alcohol levels, needing more connectivity, real-time data transmission, and integration with other systems, which makes them less effective in offering prevention or continuing education to users. Furthermore, they do not contribute significantly to broader public safety or the development of city ecosystems.

The present disclosure presents a device similar to traditional breathalyzers, while also connecting advanced data processing elements to the disclosed. The present solution enables real-time monitoring and personalized feedback, making alcohol detection part of a more significant effort to improve public safety. More importantly, the data transmission capabilities and cloud infrastructure pave the way for valuable insights for urban administration. This approach provides essential data for developing informed public health policies and urban planning, promoting prevention and education initiatives, and contributing to broader public safety.

In an embodiment, the proactive data transmission capabilities enable a network of disclosed devices that communicate with the cloud safely and efficiently, providing an additional layer of security, as data is collected in real time. It alerts users to excessive consumption and imminent risks, reassuring the community at large.

Furthermore, the data collected will be transmitted to a cloud infrastructure, where it will be analysed to identify patterns for urban administration. This approach provides essential data for developing informed public health policies and urban planning, promoting prevention and education initiatives. By collecting data for urban planning, the public is more informed and aware of its broader implications.

Regarding the environmental impact of this solution, the excessive use and repair of materials are avoided through a sustainable and modular design, and biodegradable and low-consumption batteries were opted for. Furthermore, the device incorporates sustainable recharging systems to guarantee a continuous commitment to reducing the carbon footprint.

In an embodiment, when a user's alcohol level exceeds a safe threshold, a QR code is generated that links to alternative transportation options such as ridesharing platforms, taxis or public transit. This simple function encourages users to avoid driving under the influence, reducing the likelihood of accidents.

In addition to enhancing public safety, the disclosed acts as a multi-purpose communication platform. In an embodiment, a screen can be used for public service announcements, safety tips and emergency alerts, and other types of communication, such as digital advertising. From real-time weather updates to local traffic conditions or even warnings during emergencies, this device can provide information to the public. This ability to deliver targeted information and safety alerts increases public awareness, making the city's infrastructure more responsive and connected.

Furthermore, what truly sets the disclosed device apart is its data collection and analysis capability. Every interaction with the device is logged, enabling the collection of valuable data on user behaviour, environmental conditions and use patterns. This data can be analysed to provide insights far beyond the device, feeding into broader urban planning and public safety strategies.

In an embodiment, the system also comprises a Global Manager System (GMS). Unlike other solutions from the prior art, one of the functionalities of the GMS is to analyse the calculated alcohol percentage and provide alerts accordingly, indicating that user is advised not to drive with safety, if the detected alcohol value is too high.

In an embodiment, the device comprises a payment system wherein the user may have two options: a normal payment and a free pass. Additionally, the device supports various payment methods.

### BRIEF DESCRIPTION OF THE DRAWINGS

The following figures provide preferred embodiments for illustrating the disclosure and should not be seen as limiting the scope of invention.
**Figure 1****:** Exploded view representation of an embodiment of a device for determining a breath alcohol concentration of a user.
**Figure 2A****:** Representation of a perspective view of an embodiment of the disclosed device.
**Figure 2B****:** Representation of a front view of an embodiment of the disclosed device.
**Figure 2C****:** Representation of a back view of an embodiment of the disclosed device.
**Figure 3****:** Representative flowchart of the interaction between the disclosed device, the disclosed system, associated with said device, and the user.
**Figure 4****:** Representative diagram of the interaction between components of the disclosed device, and respective the disclosed system.
**Figure 5****:** Representative diagram of an embodiment of the Global Manager Server (GMS), and respective architecture.

### DETAILED DESCRIPTION

The present disclosure relates to an intelligent and interactive digital breathalyzer device and management system that enhances traditional alcohol detection methods by integrating advanced technological features designed to improve user safety and contribute to broader public safety initiatives.

In an embodiment, this device combines precise breath alcohol concentration measurement with real-time cloud-based data processing, enabling the generation of personalized feedback and educational content. In addition to BrAC detection, the breathalyzer suggests proactive solutions, such as car-sharing or alternative transportation options, to reduce the likelihood of alcohol-related accidents.

The present disclosure provides a digital breathalyzer device, system, and method for accurately determining a user's breath alcohol concentration (BrAC). This device comprises a robust frame, a high-precision breathalyzer sensor, a disposable straw dispensing mechanism, and a multifunctional tactile screen capable of presenting diverse interactive and promotional content. It integrates secure payment processing, advanced user identification methods, and dynamic QR code generation for streamlined interaction and enhanced user experience.

The present disclosure comprises extensive real-time data collection, secure transmission, and comprehensive analytics via a centralized Global Manager Server (GMS). The GMS facilitates predictive maintenance, automated calibration alerts, and detailed monitoring of device status. Additionally, it employs advanced machine learning algorithms for predictive analytics, providing personalized, context-sensitive feedback to users, including targeted educational content and proactive mobility recommendations integrated seamlessly with local transportation services.

In an embodiment, the device's interactive functionalities extend to immersive augmented reality experiences, enhancing user awareness about alcohol consumption effects. It features robust anti-vandalism measures, structural integrity enhancements, and environmental sustainability features such as photovoltaic energy integration and components. Furthermore, the system supports multi-language interactions and accessibility features, enabling universal usability across diverse demographics.

In an embodiment, real-time event analytics delivered by the GMS offer venue operators and urban administrators detailed insights into user behavior patterns, alcohol consumption trends, device usage metrics, and peak activity periods, facilitating informed decision-making and improved public safety strategies. This combination of technological features and comprehensive data analysis capabilities positions the disclosed device as a solution for enhancing individual safety, promoting responsible behavior, and significantly contributing to urban safety and smart city initiatives. The system comprises real-time analytics algorithms, enabling the continuous monitoring of alcohol consumption patterns, user interactions, and questionnaire responses aggregated at events or locations. This analytical tool provides instant actionable insights to venue operators and urban planners, aiding public safety and effective resource allocation.

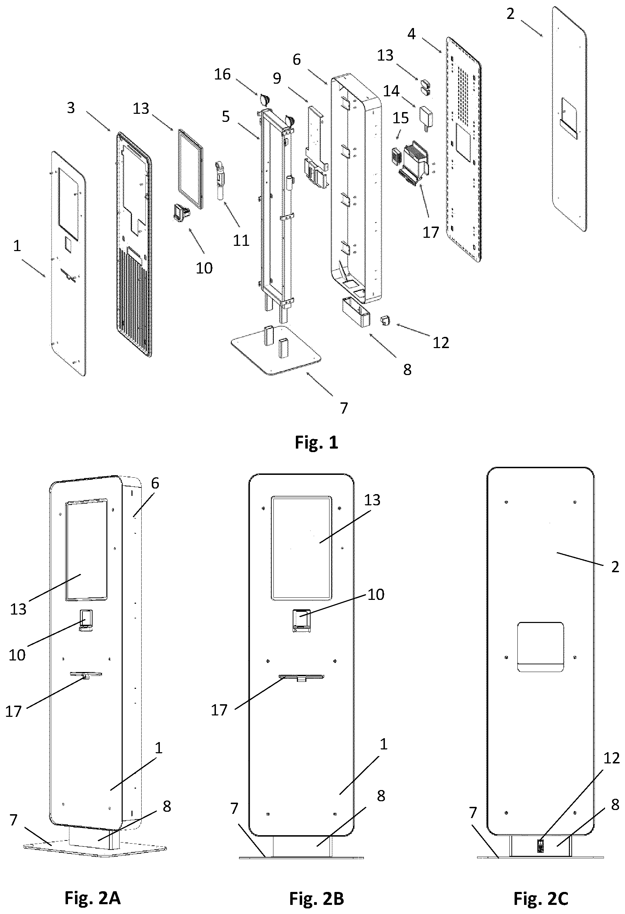

**Figure 1** represents an exploded view of an embodiment of the disclosed device for determining a breath alcohol concentration of a user, where: **1** represents a front external panel, **2** represents a back external panel, **3** represents a front LED panel, **4** represents a back LED panel, **5** represents a main structure of the device, **6** represents a frame, **7** represents a base, **8** represents a neck cover, **9** represents an inner support, **10** represents a hardware system that enables users to make payments, herein referred to as virtual payment terminal (TPV), **11** represents a blower, **12** represents an electric plug, **13** represents an electric transformer, **14** represents a computer processing unit, **15** represents a LED power supply, **16** represents a sound speaker, and **17** represents a disposable straw dispenser and a disposable straw reservoir.

**Figure 2A** represents a perspective view of an embodiment of the disclosed device, where: **1** represents a front external panel, **6** represents a frame, **7** represents a base, **8** represents a neck cover, **10** represents a hardware system that enables users to make payments, herein referred to as virtual payment terminal (TPV), **13** represents an electric transformer, and **17** represents a disposable straw dispenser and a disposable straw reservoir.

**Figure 2B** represents a front view of an embodiment of the disclosed device, where: **1** represents a front external panel, **7** represents a base, **8** represents a neck cover, **10** represents the hardware system that enables users to make payments, herein referred to as virtual payment terminal (TPV), **13** represents an electric transformer, and **17** represents a disposable straw dispenser and a disposable straw reservoir.

**Figure 2C** represents a back view of an embodiment of the disclosed device, where: **2** represents a back external panel, **7** represents a base, **8** represents a neck cover, and **12** represents an electric plug.

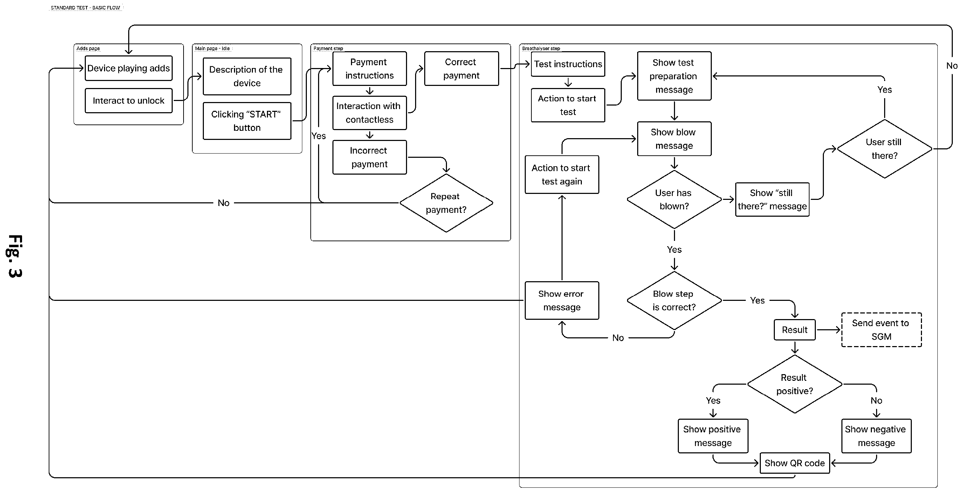

**Figure 3** represents a flowchart of an interaction between the disclosed device, the disclosed system, associated with said device, and a user. This diagram illustrates all possible interactions the user has with a breathalyzer device, in order to perform a breathalyzer test. It starts in idle or advertising mode, and proceeds through various stages: payment, test instructions, user identification, and test completion. The flowchart includes scenarios where the test may need to be repeated or where the user is not recognized.

In an embodiment, when a user approaches and interacts with the touchscreen, usually by tapping on it, the device detects the input from the user and transitions into active mode, prompting the user to start a test. If the user chooses to begin the process (often by pressing a "START" button), the device displays payment instructions, if a fee is required. At this stage, the user follows on-screen prompts to complete the payment, which may involve contactless or card transactions. Once the payment is confirmed, or alternatively, if no payment is needed for the specific configuration, the system provides instructions to said device, to conduct the breathalyzer test. These instructions may include details on how to properly position a disposable straw and the correct technique for blowing into the sensor. The user can also navigate any interactive menus, including language options and other relevant settings.

Before conducting the test, the device may attempt to identify the user by searching for an existing profile. Conversely, the device creates a new entry, if the user is unrecognized. When the user is ready, the device instructs the user to blow onto the breathalyzer. The sensor measures the alcohol content in the exhaled breath and sends this data to the computer processing unit of the device for analysis. Based on the measured value, the system determines whether the result is within acceptable limits or exceeds a legal threshold. Depending on the assessment, the device displays an appropriate message, such as "Safe to drive", "Above limit", or another relevant outcome. If the breath sample is of insufficient quality (e.g., too short or weak), the system will inform the user and request to repeat the test.

Finally, the user sees the result on the screen and may receive further instructions, such as waiting before driving or opting for an alternative form of transportation. The system can also provide a QR code for the user to scan and receive a digital ticket of their result. After a period of inactivity, or once the user has finished reviewing the outcome, the device returns to its idle state.

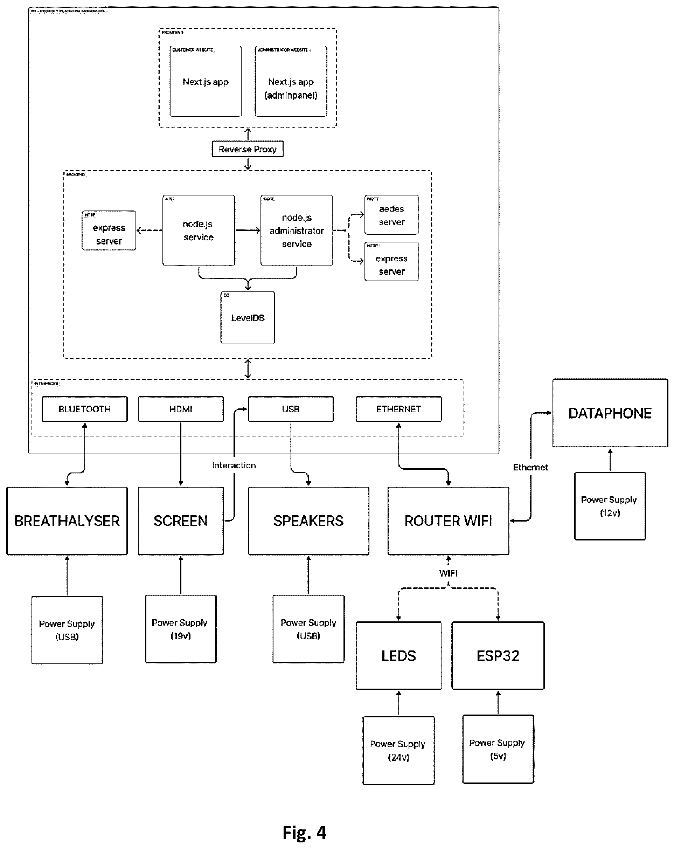

**Figure 4** describes the interaction between components of one of the disclosed devices, and the server. This diagram illustrates two major layers: the software stack at the top, and the hardware components at the bottom. The software layer consists of several key services, including two Next.js applications, one for end-users and another for administrators, as well as Node.js servers running Express, a reverse proxy, and the Aedes MQTT broker. These services communicate with each other to manage user interactions, administrative tasks, real-time data exchange, and local data storage.

In the software stack's upper section, the two Next.js applications provide distinct interfaces. The user-facing application creates an interactive environment on the device's screen, guiding individuals through the breathalyzer process and the payment procedure when necessary. In contrast, the administrator-facing Next.js application is designed for remote configuration, monitoring, and data analysis, enabling authorized personnel to manage multiple devices or calibrate tasks from a centralized location. A reverse proxy, commonly set up with an HTTP server like Nginx, routes incoming requests to the appropriate service, handling tasks such as SSL termination and load balancing.

Beneath the Next.js layers are the Node.js services. An Express server coordinates the core backend logic, receiving data from the breathalyzer sensor or other peripherals, relaying the aforementioned data to the frontend or the local database (LevelDB). Simultaneously, an additional Node.js server is dedicated to administrative functions, ensuring that sensitive operations remain separate from routine user interactions. The MQTT broker facilitates real-time communication within the device, using a lightweight publish-subscribe model. This setup is particularly effective for sending commands to the ESP32 microcontroller, controlling the LEDs, and receiving updates from various sensors without incurring significant network overhead.

In the hardware section, each component is depicted with its power supply requirements and communication interfaces. The breathalyzer sensor typically connects via USB, drawing power while measuring the blood alcohol concentration (BAC) and transmitting said data to the central processing unit. The screen, connected through HDMI, presents user instructions and results, while speakers (often powered by USB) provide audio feedback or instructions. A router, with an Ethernet connection to the main computer, ensures the device can communicate securely with remote servers or administrative dashboards. The payment terminal (dataphone) may connect via Ethernet or USB, and operates at 12V, allowing the system to handle contactless or card-based transactions.

LEDs provide visual status updates by turning different colors or flashing to indicate states such as "ready," "test in progress," or "payment required." These LEDs are controlled by an ESP32 microcontroller that often runs firmware (such as WLED) and receives commands through the Aedes MQTT broker. The ESP32 itself is powered by an appropriate supply voltage (often 5V or 3.3V) and can manage real-time tasks if the system expands to include additional sensors or features.

Power distribution is meticulously managed, with each peripheral (i.e., the breathalyzer, router, dataphone, LEDs, ESP32, and screen) receiving the correct voltage level. Data flows securely between the software and hardware components, utilizing USB for direct device connections, HDMI for video output, Ethernet for network access, and MQTT for lightweight, real-time messaging.

When a user approaches the device, they can view on-screen instructions and initiate a breathalyzer test. If payment is needed, the device prompts for it, processes the transaction through the dataphone, and then enables the breathalyzer function. Once the user provides a breath sample, the sensor sends the measurement data to the Node.js/Express server, which interprets it, logs it locally in LevelDB, and forwards it to any external or cloud-based services. The user then sees their test result on the screen while the LEDs visually confirm the process status.

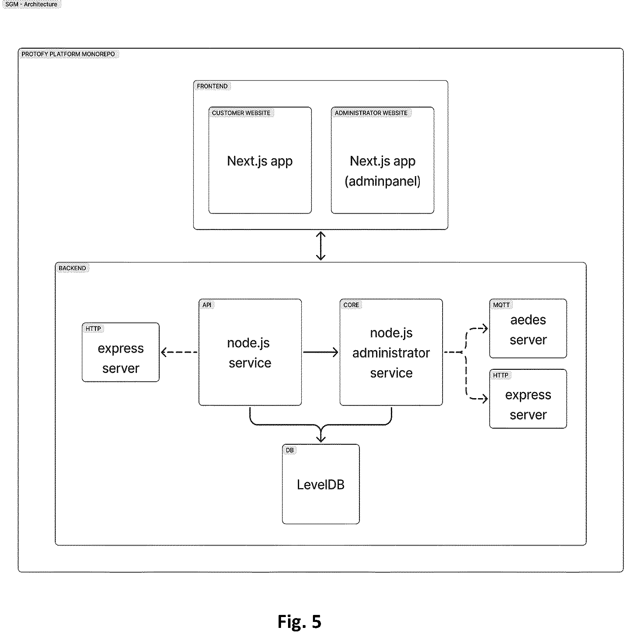

**Figure 5** depicts a diagram representation of the architecture of Global Manager Server, GMS, where two main layers, Frontend and Backend, are separated but interconnected. The frontend consists of two Next.js applications designed to provide personalized experiences: one for end-users and another for administrators. On the backend, Node.js services manage the application logic, real-time communication, and data storage. The inclusion of an MQTT broker is particularly beneficial for scenarios involving multiple loT devices, as it enables efficient, low-latency messaging. Additionally, LevelDB serves as a reliable local storage solution, enhancing the system's resilience to network interruptions.

In an embodiment, the device maintains the familiar functionality of traditional breathalyzers, using disposable straws retrieved through a container that will have an aperture inside the device, so the user can remove a disposable straw for sample collection. The breathalyser device is configured to count the number of tests that have been performed with a count cycle, thus estimating the number of disposable straws present in the disposable straw reservoir.

In an embodiment, the device also comprises a computer processing unit capable of analysing data and generating insights in real-time. This data is processed in the cloud, which provides personalized recommendations to the user, promoting responsible behaviour. Integrating this technology with loT ecosystems also allows for data aggregation, supporting public safety strategies and urban planning efforts. Similarly to traditional breathalyzers, the BrAC measurement correlates directly with alcohol concentration in the bloodstream.

In an embodiment, the device comprises a touch screen, speakers, and a vertical support. Furthermore, the device comprises shock resistance and anti-vandalism features. The device has superior durability, allowing shocks without guaranteeing its reliability, and the integration of anti-vandalism solutions, such as special screws. This way, the device provides a safe and interactive environment for tests and other interactions with a user, as described below.

In one embodiment, the touch screen has at least 0.0254 meters and 4K resolution.

In an embodiment, the device comprises a contactless system and QR codes to allow for the user to pay for the BrAC tests or services provided by third parties. This makes the device highly interactive and attractive for users who value agile and digital solutions. Furthermore, the device has a Bluetooth connection for crossing of information - sensor data retrieved by the ESP32 microcontroller - with other mobile devices (e.g., user devices, such as mobile phones or computers), and speakers for direct interaction with the user through sound feedback.

One of the most compelling strengths of the device is its potential for continuous evolution. The system is designed to be modular and adaptable, allowing for possible upgrades and additional functionalities. The main components are described below.

In an embodiment, the user can activate the device with one of three different options - contactless interaction, QR Code scanning, or NFC scanning. After activation, the device displays instructions for the alcohol test, requiring the user to start the test by clicking "OK." After starting, the user must remove one straw from the bottom of the device, insert it into the hole, and wait for the device to display a message allowing the user to exhale to the disposable straw. If the user does not want to use a straw, they can start the test and exhale directly into the hole. During the test, the device displays a loading bar that varies between 0 and 100%, displaying at the end the user's blood alcohol level. Depending on the alcohol level in the blood, the device presents a color coding (green for safety, yellow for caution, and red for danger) aligned with the colors displayed by the existing LEDs, helping users interpret their test results. Upon completion of the test, a QR code containing the test information is swiftly generated, providing the user with a convenient way to store and share the results. Additionally, an electronic receipt, complete with all the relevant details, is promptly printed, ensuring that the user has a comprehensive record of the test. Simultaneously, the system stores all information (e.g., blood alcohol level, testing location, time, among others) and prepares for a new test. To perform a new test, the user must simply remove the used straw, if any was used, and the device will be ready for the next test.

In an embodiment, the Global Manager Server (GMS) is the central component, monitoring the entire network of breathalyzer devices disclosed herein, playing a fundamental role in managing said devices. This advanced system allows complete control, such as giving orders to the digital devices disclosed herein and a detailed analysis of all network devices' actions, providing crucial real-time information. It is possible to manage all device functionalities through the GMS, from commands to specific monitoring performance, ensuring operational efficiency and quick response capacity to any situation. This tool allows for remote and effective business management, ensuring that all operational aspects are synchronized and optimized for better system performance.

In an embodiment, the system incorporates a machine learning model capable of autonomously analysing sensor drift, usage patterns, and environmental conditions to proactively predict the optimal calibration periods, thus reducing maintenance requirements and enhancing sensor reliability and accuracy.

In another embodiment, the device comprises machine learning model specifically developed to autonomously predict optimal sensor calibration intervals, ensuring peak accuracy and reliability. Said model continuously collects data on sensor drift by monitoring changes in sensor readings over time, which are compared to baseline calibration values. It also analyses historical usage patterns, including frequency of use, duration, and intensity of sensor operations. Furthermore, environmental condition data such as ambient temperature, humidity, air pressure, and altitude are gathered through integrated environmental sensors. The aforementioned measurement dataset is processed using supervised and unsupervised machine learning algorithms, such as Random Forest, Gradient Boosting Machines (GBM), and clustering algorithms like K-means clustering. Predictive models are trained periodically using historical calibration data and validated with real-time sensor performance metrics. The trained models produce forecasts on when the sensor calibration should be performed. Automated notifications and actionable alerts regarding upcoming calibration schedules are sent to administrators and maintenance personnel via the GMS, reducing unplanned downtime and enhancing overall system performance and maintenance efficiency.

The User Interface (UI), which is displayed on the screen of the breathalyzer device, is designed to focus on the user, facilitating interaction and operation of the device. The UI bridges the intelligent digital breathalyzer and the user, offering intuitive navigation and an enriching and user experience. The interface is designed to be user-friendly and accessible, allowing users to activate and use the device easily. Furthermore, the software is adaptable and can be updated and customized to meet the specific needs of the users and the market. This interface improves the user experience and provides a direct communication channel with customers, offering personalized information, feedback and interactions.

In an embodiment, the UI also includes an optional feature of quick and interactive questionnaires that appear on the screen when selected. Key features of this system are:
Enhanced interaction - The quick and interactive questionnaires increase user interaction with the device;
Valuable data collection - It collects critical insights into user behaviours and preferences;
Short and quick responses - Designed to be brief, the quiz allows users to respond quickly through a touch screen;
Dynamic interaction - Questionnaires disappear after a few seconds, creating a dynamic and spontaneous interaction;
Real-time data for third parties - The quiz captures diverse preferences in real-time, providing valuable data to companies, brands, or partners; and
High engagement rates - Placed in high-traffic environments, the device ensures higher viewing and conversion rates than traditional digital marketing.

The system offers the ability to alter any type of information according to the type of event. Therefore, the system permits the addition, management or removal of any questionnaire from any given device, and sets of answers, images, videos, and other types of information from said devices. This can be done before, during, or after the alcohol test, allowing for complete management, customization and adaptability to the specific needs of the event or location.

In an embodiment, the system comprises real-time analytics capabilities that are powered by data processing and analytics algorithms running on the GMS. The server continuously collects data streams from multiple devices, capturing detailed information on alcohol consumption results, user interactions, questionnaire responses, device location, and timing metrics. Using real-time analytics techniques, such as Complex Event Processing (CEP), anomaly detection algorithms, and streaming analytics frameworks, the system identifies significant behavioural trends and patterns among crowds and events instantly. The platform aggregates data in real-time, employing statistical models, correlation analysis, and predictive analytics to deliver actionable insights to venue operators, public safety officials, and urban planners. These insights are visualized through a dashboard that clearly illustrates key metrics such as average alcohol consumption levels, peak testing hours, user interaction intensity, and questionnaire response trends. The analytics module continuously refines its predictive capabilities through iterative learning from incoming data streams, ensuring accurate forecasting and enabling precise, real-time resource allocation and risk mitigation strategies, enhancing public safety and effective management of events.

In an embodiment, there are three forms of advertising that may be displayed in the screen of the device: audiovisual content, e.g., video with the option for the customer to choose the duration of the display; interactive content that will be displayed from the scan of a QR code displayed by the user interface (UI), or video content without sound or any other assisting medium. Images and/or videos added for advertisement, are managed through the GMS file system. Additionally, interaction with users occurs through a question tag, where both the question and the answer options are defined in the GMS. This system also records the selections made by the end user. These questions serve as a way to engage with the user, while advertising and mediating the videos presented.

In another embodiment, the UI introduces a live voting system featuring a floating widget on the screen that presents voting options to users, such as "Which music do you prefer in a nightclub: A: House, B: Latino, C: Hip-Hop?" and users can vote through a web link on the device. The voting results are updated and displayed in real-time as percentages, which creates a dynamic interaction, increasing user engagement with the device. This add-on serves as an entertainment tool and a means to gather user preference data, offering valuable insights for personalizing the user experience in the establishment.

In an embodiment, the GMS acts as the brain of the disclosed system, enabling total remote control and is essential for the efficient management of the network of smart digital breathalyzer devices. This system offers a real-time view and absolute total control over the performance of the devices, enabling an immediate and effective response to any eventuality. It provides real-time information on the status and performance of the devices, allows any interaction with the system, having the ability to add, remove, or change any types of contents and information that pass through our systems at any time, thus controlling all systems and devices, also enabling quick and effective responses to different situations.

In an embodiment, there are two different software that interact with each other, one aimed at the user UI and the other focused on the operational management of the machine universe. The GMS acts as the monitor of the system, and the UI acts as the interface with the user. The clear separation of these functions is crucial to ensure efficient operation and an exemplary user experience.

In an embodiment, the software of the GMS is the centralized management tool that will allow for the management of all operations, monitoring, and managing the network of smart breathalyzer devices. This software will be responsible for:
Providing a complete control panel for monitoring the detailed state and effectiveness of each device in real-time;
Allowing remote interaction of all kinds of orders and commands, contents, everything that happens with the device and software, solving potential technical problems, among others; and
Analysing and displaying operational data in real-time to optimize the performance and distribution of devices, among others.

In an embodiment, developing software or an application that allows total remote control of the breathalyzer devices is essential. This software should be accessible through an online platform or a dedicated control panel, providing a global view of the detailed status of each device and total interaction capability with all devices and systems.

In an embodiment, the system provides detailed information about the status of each breathalyzer device in real time, including all interactions with the device, financial control of all devices, billing per unit for each payment method, device status (on/off), product status (operational/anomalies), payment currency type (e.g.,and disposable straw reservoir in the dispenser for replenishment, alcohol sensor calibration status, uptime, among others. The system must keep a complete record of the entire payment system that occurs on the device so that one can have a detailed understanding of all device activities. In an embodiment, the system informs how much one bills with each device in real time, and how revenues are generated in detail.

In an embodiment, one must have the capacity to choose, change, or delete the advertising displayed on the screens remotely, in real time, and individually, controlling all functionalities related to the content, the duration of each advertisement, the number of repetitions of each advertisement, playlist definition, among others. The type of advertising can be in video format, image format, video streaming format, presentation of alert/awareness messages, presentation of dynamic QR codes, overlay of other images/videos over the main presentation, definition of the location and size of this overlaid presentation. It should also be possible to add tactile buttons on the screen, which allow changing the natural flow of the system, presenting specific videos, images, or QR codes, among others. These buttons should also be configurable in terms of size, position, text, color, and when they should be presented.

In an embodiment, it should be possible to define in the remote-control system what to present in various cases of different alcohol levels beyond the result value. This makes it possible to configure, for each event, what message/video/image/QR code/information can be presented based on the test result or other factors.

In an embodiment, it is vital and necessary to have access to a menu/option that allows analysing, viewing, managing, and extracting the data collected based on filters to define all the collected parameters. Detailed total record and financial control of all transactions and actions on the device are required, which are essential to maintain the financial and operational integrity of the system. The possibility to change the monetary values for using the system regardless of the payment system, and to add or remove payment systems, is considered.

In an embodiment, a comprehensive digital record of all activities of each device through the GMS is maintained. This is essential for operational, financial, and service security control. The system will provide a detailed view of the performance of each device in real time, ensuring transparency and effectiveness in service management. Furthermore, a control checks all system information, battery level of each device, ability to activate or deactivate any device remotely for energy saving and efficient management, among others, exists.

In an embodiment, the system should automatically detect and notify in real-time any anomalies or issues that arise in the breathalyzers. This includes alerts related to operational failures, calibration errors, or any situation that could affect the device's functioning. This may include a vandalism alert to the device.

In an embodiment, the system should automatically detect and notify in real-time any anomalies or issues that arise in the breathalyzer devices. This includes alerts related to operational failures, calibration errors, or any situation that could affect the device's functioning. This may include a vandalism alert to the device.

**Figure 4** represents a flowchart of the interaction between the disclosed device, the disclosed system, associated with said device, and the user.

In an embodiment, the disclosed device operates in two main modes. The device is in Idle Mode, when not in use, and the screen displays configurable advertising content from the server. When in active mode, the disclosed device is activated when the user interacts with the touchscreen to start the test.

Regarding the usage flow, the device starts in idle mod. Here, the user approaches the device. If the system is in idle mode, the user interacts with the screen. The touchscreen detects the interaction and wakes up the system, accessing the main screen (active Mode). With this, the device is in active mode, where instructions are presented and the test is initialized. If desired, the user can change the language. Afterwards, the interface displays test instructions, and when the user is ready to start the test, they press the Start button. Afterwards, the payment process begins, if enabled. Following the on-screen instructions, the user makes a payment via the Dataphone. Upon payment confirmation, the system allows the user to proceed, to the test preparation. Here, the user takes a straw from the dispenser and inserts the straw into the measurement sensor slot. The alcohol measurement starts when the user blows into the breathalyzer sensor using the straw and the sensor measures the alcohol concentration in the exhaled air and sends the result to the computer data processor. In an event generation, the system generates an event with the results and sends it to the SGM server for storage and analysis. The test results are then displayed. The information is displayed on the screen for the user to see. The user scans a QR code to receive a digital ticket. Finally, the device returns to idle mode. After a few seconds, the system resets the screen with instructions to start the test. After a period of inactivity, the system returns to idle mode and displays advertising videos.

In an embodiment, the system for determining a breath alcohol concentration of a user, comprises one or more devices for determining the breath alcohol concentration of a user, according to the disclosure, and a Global Manager Server, GMS, which is configured to:
receive the breath alcohol concentration of the user from the breathalyzer from one of the one or more devices;
send promotional and/or interactive content to the one or more devices, based on the detected breath alcohol concentration;
send an alert to each of the one or more devices to perform breathalyzer calibration;
calculate the number of tests carried out by each of the one or more devices, and send a notification from one of the one or more devise to the Global Manager Server, GMS, regarding a low number of disposable straws in the disposable straw reservoir;
change advertising content for each of the one or more devices, based on the type and duration of content;
present daily information for each of the one or more devices;
generate one or more reports and/or dashboard views regarding the events and/or the breathalyzer tests carried out by each of the one or more devices, wherein each report comprises a location of the device, an average breath alcohol concentration of various user per event, a number of tests done per event, an average time between tests, and a total paid amount per event;
manage, i.e., include and/or remove advertising content, e.g., photos and videos, by means of an integrated file system;
identify and validate the currency payment type (e.g., payment in US dollars, in British pounds, in European euros, without the need of currency, etc.) made by the user during the test;
detect a correct execution of the test
activate and deactivate the system; and
automatically adjust to the colors displayed by the LED system.

In an embodiment, the device sends the breathalyzer test results to the server. Afterwards, if the breath alcohol concentration (BrAC) is higher than a given value, the server sends a command to the computer data processor of the device a message to change the color of the lights of said device, changing from the predefined lighting color to a red color.

In an embodiment, if the user incorrectly performs a breathalyzer test, the GMS indicates to said user that test was incorrectly performed and notifies that the test should be repeated by said user. The user will receive clear instructions on correctly repeating the test, such as "Please take a deep breath and blow steadily into the device until you see the progress bar at 100%." The system will also provide visual or audio (through the User Interface) cues to guide the user through the process and ensure an accurate reading.

In an embodiment, a GPS Tracking system for various eventualities, such as location and thefts, is present. In this manner, the GMS comprises a mapping app that obtains the real-time geographic location of all devices. The GPS coordinate location can be matched with the IP address of each breathalyzer device connected to the Wi-Fi network, since the disclosed breathalyzer device comprises a router or an access point.

In an embodiment, the alcohol test display, integrated into the UI, provides a layout with information about the test and communication with the user. This layout includes five boxes: Main Advertisement Publicity/Digital Marketing; information about the Alcohol Test; Secondary Publicity and QR Codes; Complementary Information; and Motion Background. The first box is designated for the main advertisement content, displaying primary advertising and digital marketing materials, ensuring they effectively capture the audience's attention. This could include video ads, brand promotions, and key marketing messages. The second displays all relevant information regarding the alcohol test.

In an embodiment, the breathalyzer results are accurately displayed in two decimal places. The unit of measurement used is mg/L (milligram of alcohol per liter of exhaled air), which is widely recognized and standard in devices of this type. The device offers fast and reliable reading, with levels of detail that ensure accuracy in indicative measurements.

Moreover, the second box informs users about the alcohol test process, results interpretation, and safety guidelines, ensuring they are well-informed about the test they are undertaking. This enhances the overall user experience and trust in the device.

In an embodiment, the Secondary Publicity and QR codes box is used for secondary advertising and displaying QR codes to provide additional small advertising opportunities and interactive elements such as QR codes for quick access to promotions or further information. With this secondary display, it is possible to increase engagement by offering interactive elements, such as mobile applications and more advertising slots. An example would be a prize system to encourage using the intelligent digital breathalyzer and create an interactive user experience. After each breathalyzer test, the device generates a unique QR code on the screen. Users can register this QR code in the app to enter a prize draw at the end of the event. Each QR code is unique and valid only for that specific event. The QR codes can also be used for charity through a unique donation feature. Users can donate through a contactless card or QR code displayed on the device when the function is activated. Users can also make their donations public or anonymous, creating a secondary layer of data protection.

The Complementary Information box provides supplementary information relevant to the event or venue that can be updated and added. It can display details about the event schedules, social media updates, public messages, and other important announcements. Moreover, it keeps users informed and engaged with real-time updates, enhancing their experience at the venue. Finally, the Motion Background is a dynamic area that adds visual appeal. It creates a visually stimulating environment that complements the static content displayed in other sections. This area enhances the overall aesthetic of the display, making it more attractive and engaging for viewers.

Moreover, the intelligent digital breathalyzer provides accurate results on screen after each test. Accuracy is a priority, where the disclosed device is capable of performing thousands of tests without recalibration, maintaining a high level of accuracy. The breathalyzer device is specially designed for busy events, offering quick results (in approximately 3 seconds) and contributing to safety and accident prevention. Furthermore, the GSM provides a calibration detection mechanism that identifies when the device needs to be calibrated as a function of the number of breathalyser tests performed and the affluence of any given event. This ensures the maintenance of the system' accuracy and reliability of results, minimizing the need for frequent manual recalibrations, since the system will collect all the necessary information to calibrate the device, especially in challenging, high-traffic environments.

The system enables the display of videos, images, QR codes, and content overlay, creating an interactive and engaging experience. The content is highly customizable and controllable in real-time, allowing quick content adaptation. The GM system guarantees total control and flexibility in managing the content displayed. Furthermore, the system offers the possibility of activating touch screens as needed.

An embodiment comprises an analysis system crucial to providing valuable information about the device's interactions with users and the environment. This system, integrated within GSM, provides insights on the number of adds shown, the number of events, and number tests performed during an event. This system collects and analyses the interaction data of each breathalyzer device, supporting strategic decisions and creating business opportunities. Moreover, GMS applies potential Al integration to generate automatic reports, detect data patterns, generate intelligent alerts, and extract advanced metrics on system usage, improving decision-making and service optimization. The device can offer preventative recommendations tailored to each situation based on individual and contextual parameters, promoting a proactive approach to safety and responsible consumption.

In an embodiment, in addition to alcohol testing, the device works as an interactive tool for communication, research and marketing, generating valuable data on preferences and user behaviours. This component will transform raw data, the raw data comprising breathalyzer device test information - date, alcohol level, local - and event information - duration, geographic location, number of tests; etc. - into actionable insights, allowing the system to refine the user experience and enhance the device's functionality. The collected data helps optimize the sale of valuable databases, distribution of devices, customization of marketing campaigns, and development of strategic partnerships, among other things.

In an embodiment, the smart breathalyzer comprises an identification (ID) scanner capable of reading various forms of identification - ID Verification and Incident Reporting System - such as driver's licenses, ID cards and passports. This functionality allows for real-time verification of age and identity, ensuring compliance with legal requirements and enhancing overall security.

The software architecture is built as a set of independent microservices communicating via Hypertext Transfer Protocol (HTTP/S) application programming interfaces (APIs) and a messaging bus. The microservices emit events on the bus and make point-to-point calls between them. Moreover, the proposed smart breathalyzer relies on Cloud APIs developed in JavaScript/TypeScript using Node.js and Express. In development or standalone mode, it uses LevelDB for persistence but can be configured to use external persistence. This enables horizontal scalability in the cloud by creating stateless API compute nodes using shared storage like DynamoDB.

Message Queuing Telemetry Transport (MQTT), via the Aedes Node Package Manager (NPM) package, is used for decoupled communication between system elements. It scales well vertically but can be replaced with a cloud-provided messaging system if it exposes an MQTT interface.

The User Interface was developed in React, built with Tamagui (compatible with React Native and React DOM), and packaged as an NPM library to be as agnostic as possible to the frontend infrastructure. It can be exposed via a native mobile app, an Electron app, or a Next.js frontend.

The system uses a single language (TypeScript/JavaScript) with shared local NPM libraries for standard parts, such as object definitions and validators. All components are deployable via Docker, with hot reload, development and production modes, and environment-based configurations.

To ensure interoperability and scalability, the system uses Pino for logging, sending logs to files, and via MQTT, allowing it to integrate with any logging collection system like ELK.

In an embodiment, the screen is a touchscreen for primary user interaction interface, and the breathalyzer sensor is a high-precision alcohol measurement technology. The payment system allows card payments, with the POS terminal by Ingenico, the payment processing software by SIBS, and the library enabling integration between the Ingenico POS terminal and the SIBS software solution is the Ingelink Library by SVDI-Retail. The LEDs provide visual feedback on process status, and the speakers are used for auditory instructions, audio playback, and notifications. A microcontroller (ESP32) controls the LEDs via open-source WLED firmware, and receives commands from the system (software platform on the computer). The computer data processor is a central processing unit, which connects and manages peripherals. A WiFi Router enables the mini PC's internet connection and communication with the SGM. The device contains power supplies of 5V, 12V, 19V and 24V.

In an embodiment, the breathalyzer sensor is equipped with "SuraCell" technology (an electrochemical alcohol sensor), that offers the option to measure alcohol levels using both alcohol detection mode and alcohol measurement mode, utilizing a highly durable electrochemical sensor. This breathalyzer also retains the fast measurement speed, accuracy, LED indicator functionality, measurement result storage, and calibration notification service, while also introducing wireless communication and rechargeable battery capabilities.

In an embodiment, the mechanical components comprise a disposable straw dispenser, for automatic system for dispensing disposable straws, and a casing, which is a physical design of the device, integrating various hardware elements.

In an embodiment, the SGM is a server responsible for storing and managing device usage data.

It is also important to note that this solution addresses a critical opportunity in the urban security market for municipalities, event organizers, companies and transport services. With growing concerns about alcohol-related incidents in public spaces, there is a global demand for practical solutions that promote responsible drinking and improve public safety. Thus, this smart breathalyzer offers municipalities and cities a proactive tool for real-time monitoring, allowing them to make data-driven decisions and allocate resources more efficiently. Event organizers - the main Application Scenario - can use this system to manage alcohol consumption during festivals and night-time events, reducing risks and ensuring a safer environment for attendees. By combining public safety with advanced technology, our solution has significant and highly scalable market potential, promoting healthier urban environments.

The described solution is also designed for high scalability, expanding across multiple urban environments and adapting to different use cases. As cities grow, the system can be scaled by incorporating more physical units (e.g. more alcohol meters) and expanding its data processing capabilities in the Cloud and on local devices (e.g. edge layer), ensuring that real-time analysis remains efficient. Furthermore, the interoperability of our solution allows for seamless integration with other smart city technologies, such as traffic management systems and public transport networks.

This adaptability ensures that the disclosed device and system can be adapted to meet the specific needs of different contexts, promoting widespread adoption. Ultimately, the solution is also designed to evolve with urban changes, making it a sustainable choice, capable of improving public safety in diverse environments.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

### References

[1] Flango, V. E., & Cheesman, F. L. (2009). Effectiveness of the SCRAM alcohol monitoring device: A preliminary test. Drug Court Review, 6(2), 109-134
[2] Li, B., Downen, R. S., Dong, Q., Tran, N., LeSaux, M., Meltzer, A. C., & Li, Z. (2020). A discreet wearable iot sensor for continuous transdermal alcohol monitoring-Challenges and opportunities. IEEE sensors journal, 21(4), 5322-5330.
[3] Khemtonglang, K., Chaiyaphet, N., Kumsaen, T., Chaiyachati, C., & Chuchuen, O. (2022). A smart wristband integrated with an loT-based alarming system for real-time sweat alcohol monitoring. Sensors, 22(17), 6435.
[4] Schechtman, E., & Shinar, D. (2011). An analysis of alcohol breath tests results with portable and desktop breath testers as surrogates of blood alcohol levels. Accident Analysis & Prevention, 43(6), 2188-2194.
[5] Seco, I., Gaspar, P. D., Magrinho, M., & Castelo-Branco, M. (2018, December). Preliminary study of a non-invasive portable device for continuous monitoring of blood alcohol concentration. In 2018 12th International Conference on Sensing Technology (ICST) (pp. 375-381). IEEE.
[6] Koch, K., Maritsch, M., Van Weenen, E., Feuerriegel, S., Pfäffli, M., Fleisch, E., ... & Wortmann, F. (2023, April). Leveraging driver vehicle and environment interaction: Machine learning using driver monitoring cameras to detect drunk driving. In Proceedings of the 2023 CHI Conference on Human Factors in Computing Systems (pp. 1-32).
[7] Syed, A. S., Sierra-Sosa, D., Kumar, A., & Elmaghraby, A. (2021). IoT in smart cities: A survey of technologies, practices and challenges. Smart Cities, 4(2), 429-475.
[8] Neville, F. G., Williams, D. J., Goodall, C. A., Murer, J. S., & Donnelly, P. D. (2013). An experimental trial exploring the impact of continuous transdermal alcohol monitoring upon alcohol consumption in a cohort of male students. PLoS One, 8(6), e67386.
[9] Jalal, A. H., Arbabi, S., Ahad, M. A., Alam, F., & Ahmed, M. A. (2024). Wearable Alcohol Monitoring Device for the Data-Driven Transcutaneous Alcohol Diffusion Model. Sensors, 24(13), 4233.
[10] Song, H., Pan, M., & Chen, Y. (2016). Nightlife and public spaces in urban villages: A case study of the Pearl River Delta in China. Habitat International, 57, 187-204.
[11] Friedewald, M., Da Costa, O., Punie, Y., Alahuhta, P., & Heinonen, S. (2005). Perspectives of ambient intelligence in the home environment. Telematics and informatics, 22(3), 221-238.
[12] Tornikoski, D. (2024). Service productization: service productization for a night club business.
[13] Morosan, C., & DeFranco, A. (2014). When tradition meets the new technology: An examination of the antecedents of attitudes and intentions to use mobile devices in private clubs. International Journal of Hospitality Management, 42, 126-136.
[14] Rui, L., Li, K., Jiang, M., & Jiang, X. (2024). Exploring the Factors Influencing Tourists 'Satisfaction and Continuance Intention of Digital Nightscape Tour: Integrating the Design Dimensions and the UTAUT2. Sustainability, 16(22), 9932.

## Claims

1. Device for determining a breath alcohol concentration of a user comprising:
a frame for supporting the device;
a breathalyzer for detecting the breath alcohol concentration of the user;
a disposable straw dispenser attached to the frame, for dispensing a disposable straw to perform the breath alcohol concentration detecting;
a disposable straw reservoir containing disposable straws for feeding the disposable straw dispenser with straws;
an aperture attached to the frame and connected to the breathalyzer for receiving the disposable straw for the user to blow onto;
a tactile screen attached to the frame for displaying information to the user and for the user to input information;
a payment terminal attached to the frame for receiving payments; and
a computer processing unit connected to the breathalyzer, the disposable straw dispenser,
the tactile screen and the payment processor, wherein said computer processing unit is configured to:
receive payment data from the payment terminal,
dispense the disposable straw from the disposable straw reservoir to the disposable straw dispenser,
acquire the breath alcohol concentration from the breathalyzer,
display the acquired breath alcohol concentration on the tactile screen, and
display promotional and/or interactive content on the tactile screen based on the detected breath alcohol concentration.

2. Device according to the previous claim, further comprising a card reader for receiving an identity card of the user, wherein said card reader is electronically connected to the computer processing unit for identifying the user for which the acquired breath alcohol concentration is made, preferably wherein the card reader is configured for receiving a driver's license, a government issued identification card and/or a passport.

3. Device according to any of the previous claims, further comprising:
a wireless internet router for transmitting information through the internet to a Global Manager Server, GMS, of the acquired breath alcohol concentration and, optionally, the user identification; and/or
a Global Positioning, GPS, tracker, for tracking the location of the device.

4. Device according to any of the previous claims, further comprising a plurality of lights wherein the plurality of lights can be selected from: LEDs, incandescent lights or florescent lights.

5. Device according to the previous claim, further comprising a microcontroller data processor configured to change the color and/or intensity of the plurality of lights, and wherein the computer processing unit is further configured to send lighting commands to the microcontroller data processor, wherein said lighting commands include adjusting the color of the plurality of lights, and/or the intensity of the plurality of lights.

6. Device according to any of the previous claims, wherein the computer processing unit is also configured:
to calibrate the breathalyzer;
to count the number of disposable straws in the disposable straw reservoir; and
to send an alert to the Global Manager Server, GMS whenever the disposable straw reservoir contains a low amount of disposable straws.

7. Device according to any of the previous claims, further comprising one or more sound speakers, connected to the computer processing unit, for emitting sound and music in respect to the promotional and/or interactive content, and/or wherein the computer processing unit is configured for sending sound messages and/or sound recordings to the one or more sound speakers, synchronously with the displayed promotional and/or interactive content on the tactile screen.

8. Device according to any of the previous claims, wherein the displayed promotional and/or interactive content may be selected from: a questionnaire for the user to answer, a disclaimer regarding driving under the influence of alcohol, and a promotional content.

9. Device according to any of the previous claims, wherein the payment terminal is a contactless payment terminal.

10. System for determining a breath alcohol concentration of a user, comprising:
one or more devices for determining a breath alcohol concentration of a user, according to any of the previous claims; and
a Global Manager Server, GMS, configured to:
receive the breath alcohol concentration of the user from the breathalyzer from one of the one or more devices,
send promotional and/or interactive content to the one or more devices, based on the detected breath alcohol concentration,
send an alert to each of the one or more devices, to perform breathalyzer calibration, and validate the currency payment type made by the user during the test.

11. Method for determining a breath alcohol concentration of a user, comprising:
sending the breath alcohol concentration from the breathlyzer of one of the one or more devices to the computer processing unit of the one of the one or more devices;
forwarding the sent breath alcohol concentration from the computer processing unit of the one of the one or more devices to the Global Manager Server, GMS;
processing the forwarded breath alcohol concentration by the GMS; and
registering the processed breath alcohol concentration by the GMS.

12. Method according to the previous claim, wherein communication with the GMS is by Message Queuing Telemetry Transport (MQTT) protocol, the method comprising:
using a publisher/subscriber model, in which the computer processing unit of said one or more devices acts as a publisher;
sending the breath alcohol concentration data to a specific topic on a MQTT broker;
the GMS subscribing to the specific topic for receiving data in real-time; and
processing the forwarded breath alcohol concentration by:
extracting the forwarded breath alcohol concentration, and any associated metadata, from the one or more devices,
transforming the extracted data for uniform data formatting, anomaly removal, or adding context, in particular user identification, time stamps, and
loading into the GMS the transformed data.

13. A computer processing unit configured for carrying out the steps of the method according to any of the claims 11 or 12.

14. A computer program comprising instructions which, when the program is executed by a computer, cause the computer to carry out the steps of the method according to any of the claims 11 or 12.

15. A computer-readable storage medium comprising instructions which, when executed by a computer, cause the computer to carry out the steps of the method according to any of the claims 11 or 12, in particular the computer-readable storage medium being a non-transitory computer-readable storage medium.
